# EUROPEAN PATENT SPECIFICATION

(11) **EP 1 453 452 B1**
(45) Date of publication and mention of the grant of the patent: **18.01.2012**
(21) Application number: 02789965.7
(22) Date of filing: 03.12.2002
(51) Int. Cl.: A61F 13/15, B29C 65/00, H05B 6/02

(54) **DISPOSABLE ABSORBENT ARTICLE INCLUDING ELECTROMAGNETIC BOND AND METHOD FOR PRODUCING IT**
ABSORBIERENDER WEGWERFARTIKEL MIT ELEKTROMAGNETISCHER VERBINDUNG UND HERSTELLUNGSVERFAHREN DAFÜR
ARTICLE ABSORBANT JETABLE COMPRENANT UNE LIAISON ELECTROMAGNETIQUE ET SON PROCEDE DE PRODUCTION

(30) Priority: 14.12.2001 US 340556 P
(43) Date of publication of application: 08.09.2004
(73) Proprietor: THE PROCTER & GAMBLE COMPANY, Cincinnati, OH 45202 (US)
(72) Inventor: ANGSTADT, John, Joseph, Cincinnati, OH 45211 (US); GIBSON, Frederick, William, Liberty Township, OH 45044 (US); WALSH, Bradley, Edward, Cincinnati, OH 45240 (US)
(74) Representative: Heide, Ute
(86) International application number: PCT/US2002/038382
(87) International publication number: WO 2003/051255

(56) References cited:
- WO-A-99/44559
- US-A- 4 190 010
- US-A- 6 024 822

## Description

### FIELD OF THE INVENTION

The present invention relates to a disposable absorbent article including a fusion bond accomplished by the application of an electromagnetic field and a method for producing the fusion bond.

### BACKGROUND

Disposable absorbent articles including baby diapers, baby pull-on diapers, adult incontinence articles, and feminine hygiene articles, are well known articles of manufacture intended to absorb and contain bodily discharges. Typically, these articles are manufactured on high-speed production lines by joining or bonding the components of the articles by use of adhesive or non-adhesive-bonding techniques such as ultrasonic bonding, pressure bonding or heat bonding.

WO 99/44559 A1 discloses a method of making a flangeless seam for a disposable article.

US 6 024 822 discloses a method of utilizing microwave energy in the manufacture of disposable nonwoven absorbent articles such as diapers and feminine care products.

When a single type of bonding is not sufficient to provide the desired benefit, a combination of the above bonding techniques is typically used. For example, in the areas where a bond is subjected to a prolonged and substantial extension force during the wear of a disposable absorbent article, the typical adhesives can not be sufficient to maintain the bond over a time because typical adhesives used in the manufacture of disposable absorbent articles can creep, i.e., lose their solid bond shape, which connects the adjacent surfaces of the article, and stretch under the prolonged extension force, thus, allowing the bonded surfaces to move apart from their initial bonding position. This can result in partial or complete destruction of the bond. In order to prevent this, such adhesive bonds are typically reinforced with fusion bonds, such as ultrasonic bonds, pressure bonds, or heat bonds, which can provide direct fusion of contacting surfaces by melting the contacting surfaces into each other.

However, the fusion bonds produced by ultrasonic bonding, pressure bonding or heat bonding typically affect the outer surface(s) of the bonded components. For example, the ultrasonic bonding is typically provided by means of ultrasonic vibration tools being in direct physical contact with the outside surfaces of the bonded materials, thus, leaving tool imprints on these outside surfaces. The imprints can have hard, raspy protuberances that can be irritating to the skin. Further, the pressure bonding uses compression tools applied to the outside surfaces of the bonded materials to provide pressure and velocity differential between the inside surfaces to be bonded to each other and, thus, can result in hard, raspy protuberances on the outside surfaces. Still further, the heat or thermal bonding is often accomplished by the use of hot tools entering the outside surface(s) of the bonded materials to provide heat energy sufficient to melt the inside surfaces for bonding to each other. These hot tools can also result in hard and raspy protuberances on the outside surface(s) of the bonded materials.

With respect to bonding porous materials, the heat bonding can be accomplished without the use of a hot entering tool, but rather by a hot air, as disclosed, for example, in EP 0 844 062 A1, where the hot air is applied to the outside surface(s) of the materials so as to penetrate the porous materials and to heat and melt their inside surfaces for bonding to each other. However, the contact between the inside surfaces is provided by compressing the materials from the outside surfaces by a direct physical contact of the outside surfaces. Although the above hot-air method provides enhanced tactile characteristics of the outside surfaces of the bonded materials, the compressing tools can still have a negative affect on the outside surface(s) because the outside surfaces are hot from the hot air entering the bonded materials and can have undesirable imprints and hardened areas.

In other instances, it can be desirable to provide a fusion bond between already assembled selected layers or a disposable absorbent article without affecting other layers of the article. For example, it may be desirable to provide a fusion bond between layers of materials adjacent to an absorbent core of the disposable absorbent article without bonding the core to other layers of the article and without negatively affecting the core itself. (However, it should be noted that in certain instances it might be desirable to affect the core in certain positive ways resulting in improved performance of the core or the article.)

Examples of fusion bond applications can include bonding of longer elasticized side panels (also referred to as side connectors or ears) of the article to the outer casing of the article in order to provide more elasticity in holding the article around the wearer's torso. Normally, the side panels are bonded outside the core area, adjacent to the longitudinal edges of the article, thus limiting the length of the side panel extending laterally from the longitudinal edges of the article. As a result, the limited length of the side panel generally limits the degree of elasticity of the side panel. However, a longer and more elastic side panel would require extension of the side panel into the core area and fusion bonding of the side panel to the outer casing of the article directly above the core. To provide such fusion bonding by use of ultrasonic bonding, pressure bonding or heat bonding would not only result in bonding the core to other materials of the article but also would affect the absorbent core itself by bonding the materials inside the core. This can be undesirable due to several problems. Bonding the materials inside the absorbent core can damage the core, inhibiting its desired performance in absorbing and distributing bodily exudates inside the core. With respect to the fusion bonding between the absorbent core and the outer casing of the article, the problems can include leakage of the bodily exudates absorbed by the core through holes in the outer casing that can be created during the fusion bonding of the outer casing to the core. Further, fusion bonds are normally more rigid than adhesive bonds, which are more giving, and, therefore, fusion bonding of the absorbent core to the outer casing can also result in increased rigidity of the article, now including the rigidity of the core itself. This can be problematic in providing a desired flexibility and conformity of the article around the body of a wearer.

Consequently, it would be beneficial to provide a disposable absorbent article having components or materials bonded together by a fusion bond capable to withstand a prolonged extension force without creeping, wherein the outside surface of such materials has desirable tactile properties, which are minimally or substantially unaffected by the fusion bond inside the bonded materials. Further, it would be desirable to provide a disposable absorbent article having a fusion bond between adjacent components or materials of the disposable absorbent article, capable to withstand a prolonged extension force without creeping and without adversely affecting other components or materials of the article, for example, the absorbent core. Still further, it would be beneficial to provide a disposable absorbent article having a fusion bond between adjacent components or materials of the article, disposed directly above the absorbent core of the article, wherein the absorbent core is not fusion bonded to the materials above the absorbent core. Still further, it would be beneficial to provide a bonding method for manufacturing such disposable absorbent articles. Still further, it would be beneficial that such a bonding method is applicable for fusion bonding of both porous and non-porous materials. Furthermore, it would be beneficial to provide a bonding method that would be applicable in high-speed production of disposable absorbent articles.

### SUMMARY OF THE INVENTION

In response to the difficulties and problems discussed above, a new improved disposable absorbent article and a process for manufacturing such an article have been discovered.

In another aspect, the present invention concerns a disposable absorbent article as defined in claim 1.

In another aspect, the present invention concerns a method of bonding components of a disposable absorbent article as defined in claim 9.

### BRIEF DESCRIPTION SHOWN IN THE DRAWINGS

While the specification concludes with claims particularly pointing out and distinctly claiming the subject matter, which is regarded as the present invention, it is believed that the invention will be more fully understood from the following description taken in conjunction with the accompanying drawings, in which:
Figure 1 is a plan view of a disposable absorbent article of the present invention that can be produced by the method of the present invention; the article is a diaper, shown in a flat-out state, wherein the outer-facing side of the diaper is oriented towards the viewer;
Figure 2 is a cross-sectional view of the diaper in Figure 1, taken along the cut line 2-2;
Figure 3 is an enlarged cross-sectional view of a bonding area shown in Figure 2; and
Figure 4 is a cross-sectional view of one embodiment of the method of the present invention used to make a fusion bond in a disposable article of the present invention.

### DETAILED DESCRIPTION OF THE INVENTION

The present invention is directed towards disposable absorbent articles having a fusion bond, which joins two or more components or materials of the article by exposing the article to an electromagnetic field.

As used herein, the term "absorbent article" refers to devices such as baby diapers, baby swim diapers, pull-on diapers, training pants, adult incontinence articles, feminine hygiene articles and the like, which can be placed against or in proximity to the body of the wearer to absorb and contain the various exudates discharged from the body.

The term "disposable" is used herein to describe absorbent articles, which generally are not intended to be laundered or otherwise restored or reused as absorbent articles (i.e., they are intended to be discarded after a single use and, preferably, to be recycled, composted or otherwise discarded in an environmentally compatible manner).

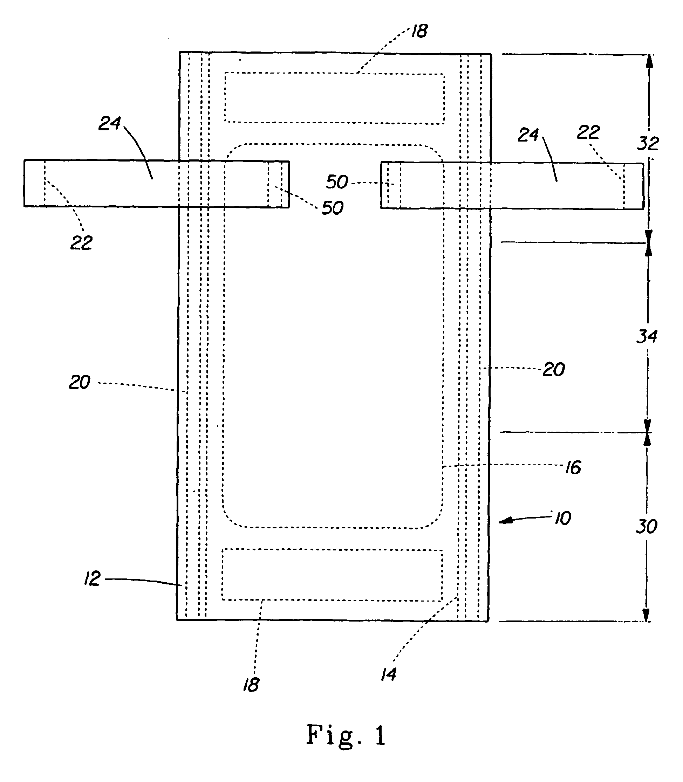

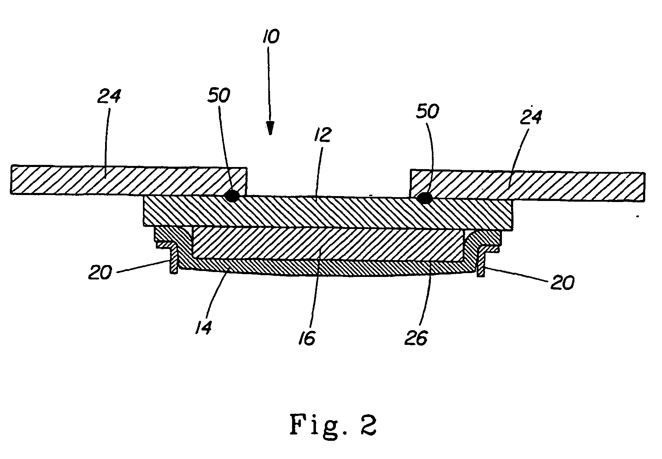

For ease of understanding, much of the following description with respect to disposable absorbent articles will be made in terms of a diaper 10 shown in Figure 1, in a flat-out, uncontracted state, having an outer cover or outer casing oriented toward the viewer, and also shown in Figure 2 in a cross-sectional view taken along line 2-2 of Figure 1. As shown in Figure 1, the diaper 20 preferably comprises a liquid impervious outer casing 12; a liquid pervious inner casing 14; an absorbent core 16 which is preferably positioned between at least a portion of the outer casing 12 and the inner casing 14; waist features 18; leg cuffs 20, fasteners 22, and side panels 24. The diaper 10 is shown in Figure 1 to have a front waist region 30, a back waist region 32 opposed to the front waist region 30, and a crotch region 34 located between the front waist region 30 and the back waist region 32. Embodiments of the present invention can also include pockets for receiving and containing waste, spacers which provide voids for waste, barriers for limiting the movement of waste in the article, compartments or voids which accept and contain waste materials deposited in the diaper 10, and the like, or any combinations thereof.

The outer casing 12, which is often referred to as a backsheet, is preferably impervious to liquids (e.g., urine) and comprises a thin plastic film such as a thermoplastic film having a thickness of about 0.012 mm (0.5 mil) to about 0.051 mm (2.0 mils). Suitable outer casing films include those manufactured by Tredegar Industries Inc. of Terre Haute, IN and sold under the trade names X15306, X10962 and X10964. Other suitable outer casing materials can include breathable materials, which permit vapors to escape from the diaper 10 while still preventing exudates from passing through the outer casing 12. Exemplary breathable materials can include materials such as woven webs, nonwoven webs, composite materials such as film-coated nonwoven webs, microporous films such as manufactured by Mitsui Toatsu Co., of Japan under the designation ESPOIR NO and by Exxon Chemical Co., of Bay City, TX, under the designation EXXAIRE, and monolithic films such as manufactured by Clopay Corporation, Cincinnati, OH under the name HYTREL blend P18-3097. Some breathable composite materials are described in greater detail in PCT Application No. WO 95/16746 published on June 22,1995 in the name of E. I. DuPont; U.S. Patent No. 5,938,648 issued on August 17, 1999 to LaVon et al.; U.S. Pat. No. 5,865,823 issued on February 2, 1999 in the name of Curro; and U.S. Pat. No. 5,571,096 issued to Dobrin et al. on November 5, 1996.

The outer casing 12, or any portion thereof, can be elastically extensible in one or more directions. In one embodiment, the outer casing 12 can comprise a structural elastic-like film ("SELF") web. A structural elastic-like film web is an extensible material that exhibits an elastic-like behavior in the direction of elongation without the use of added elastic materials and is described in more detail in U.S. Patent No. 5,518,801 entitled "Web Materials Exhibiting Elastic-Like Behavior" issued to Chappell, et al. on May 21, 1996. In alternate embodiments, the outer casing 12 can comprise elastomeric films, foams, strands, or combinations of these or other suitable materials with nonwovens or synthetic films.

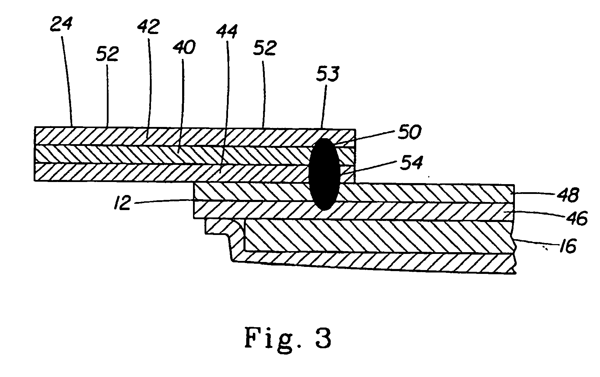

Referring to Figures 1-3, wherein Figure 3 shows an enlarged cross-sectional view of the fusion bond 50 shown in Figure 2, in preferred embodiments of the present invention, the outer casing 12 comprises a two-layer-laminate construction including a film layer 46 and a non-woven layer 48. The outer casing 12 is preferably not susceptible to an electromagnetic field. The term "not susceptible to an electromagnetic field" refers herein to any material or a substance that does not become heated or chemically active during exposure to an electromagnetic field. The term is described in more detail herein below.

The outer casing 12 can be joined to the inner casing 14 and the absorbent core 16 by any attachment means known in the art. (As used herein, the term "joined" encompasses configurations whereby an element is directly secured to another element by affixing the element directly to the other element, and configurations whereby an element is indirectly secured to another element by affixing the element to intermediate member(s), which in turn are affixed to the other element.) For example, the attachment means can include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include several lines of adhesive filaments which are swirled into a spiral pattern, as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Adhesives that have been found to be satisfactory are manufactured by H. B. Fuller Company of St. Paul, Minnesota and marketed as HL-1620 and HL-1358-XZP. Alternatively, the attachment means can comprise heat bonds, pressure bonds, ultrasonic bonds, dynamic mechanical bonds, or any other suitable attachment means or combinations of these attachment means as are known in the art.

The inner casing 14, which is often referred to as a topsheet, is preferably positioned adjacent a body surface 26 of the absorbent core 16 and can be joined thereto and/or to the outer casing 12 by any attachment means known in the art. Suitable attachment means are described above with respect to means for joining the outer casing 12 to the inner casing 14 and the core 16. In one preferred embodiment of the present invention, the inner casing 14 and the outer casing 12 are joined directly to each other in some locations and are indirectly joined together in other locations by directly joining them to one or more other elements of the diaper 10.

The inner casing 14 is preferably compliant, soft feeling, and non-irritating to the wearer's skin. Further, at least a portion of the inner casing 14 is liquid pervious, permitting liquid to readily penetrate through its thickness. A suitable inner casing can be manufactured from a wide range of materials, such as porous foams, reticulated foams, aperture plastic films, or woven or nonwoven materials of natural fibers (e.g., wood or cotton fibers), synthetic fibers (e.g., polyester or polypropylene fibers), or a combination of natural and synthetic fibers.

The absorbent core 16 can comprise any absorbent material which is generally compressible, conformable, non-irritating to the wearer's skin, and capable of absorbing and retaining liquids such as urine and other certain body exudates. The absorbent core 16 can be preferably joined to the outer casing 12 and the inner casing 14 by any suitable adhesive bonding means described hereinabove with respect to adhesive bonding means for joining the outer casing 12 to the inner casing 14. The absorbent core 16 can be manufactured in a wide variety of sizes and shapes (e.g., rectangular, hourglass, "T"-shaped, asymmetric, etc.) and can comprise a wide variety of liquid-absorbent materials commonly used in disposable diapers and other absorbent articles such as comminuted wood pulp, which is generally referred to as air felt. Examples of other suitable absorbent materials include creped cellulose wadding; melt blown polymers, including conform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials. Exemplary absorbent structures for use as the absorbent core 16 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735 entitled "High Density Absorbent Members Having Lower Density and Lower Basis Weight Acquisition Zones" issued to Alemany et al. on May 30, 1989; U.S. Patent 4,888,231 entitled "Absorbent Core Having A Dusting Layer" issued to Angstadt on December 19, 1989; U.S. Pat. No. 5,137,537 entitled "Absorbent Structure Containing Individualized, Polycarboxylic Acid Crosslinked Wood Pulp Cellulose Fibers" issued to Herron et al. on August 11, 1992; U.S. Patent 5,147,345 entitled "High Efficiency Absorbent Articles For Incontinence Management" issued to Young et al. on September 15, 1992; U.S. Pat. No. 5,342,338 entitled "Disposable Absorbent Article For Low-Viscosity Fecal Material" issued to Roe on August 30, 1994; U.S. Pat. No. 5,260,345 entitled "Absorbent Foam Materials For Aqueous Body Fluids and Absorbent Articles Containing Such Materials" issued to DesMarais et al. on November 9, 1993; U.S. Pat. No. 5,387,207 entitled "Thin-Until-Wet Absorbent Foam Materials For Aqueous Body Fluids And Process For Making Same" issued to Dyer et al. on February 7, 1995; and U.S. Pat. No. 5,625,222 entitled "Absorbent Foam Materials For Aqueous Fluids Made From High Internal Phase Emulsions Having Very High Water-To-Oil Ratios" issued to DesMarais et al. on July 22, 1997.

The elastic waist feature 18 helps to provide improved fit and containment. The elastic waist feature 18 can be joined to the inner casing 14 by any attachment means known in the art; suitable attachment means are described above with respect to means for joining the outer casing 12 to the inner casing 14. The elastic waist feature 18 is generally intended to elastically expand and contract to dynamically fit the wearer's waist. The elastic waist feature 18 can be constructed in a number of different configurations including those described in U.S. Pat. No. 4,515,595 issued to Kievit et al. on May 7, 1985; U.S. Pat. No. 4,710,189 issued to Lash on December 1, 1987; U.S. Pat. No. 5, 151,092 issued to Buell on September 9, 1992; and U.S. Pat. No. 5,221,274 issued to Buell on June 22, 1993. Other suitable waist configurations can include waistcap features such as those described in U.S. Pat. No. 5,026,364 issued to Robertson on June 25, 1991 and U.S. Pat. No. 4,816,025 issued to Foreman on March 28, 1989.

Leg cuffs 20 provide improved containment of liquids and other body exudates. The leg cuffs 20 can be joined to the inner casing 14 by any attachment means known in the art; suitable attachment means are described above with respect to means for joining the outer casing 12 to the inner casing 14. Leg cuffs 20 can also be referred to as leg bands, side flaps, barrier cuffs, or elastic cuffs. U.S. Patent 3,860,003 describes a disposable diaper which provides a contractible leg opening having a side flap and one or more elastic members to provide an elasticized leg cuff (a gasketing cuff). U.S. Patent Nos. 4,808,178 and 4,909,803 issued to Aziz et al. on February 28, 1989 and March 20, 1990, respectively, describe disposable diapers having "stand-up" elasticized flaps (barrier cuffs), which improve the containment of the leg regions. U.S. Pat. Nos. 4,695,278 and 4,795,454 issued to Lawson on September 22, 1987 and to Dragoo on January 3, 1989, respectively, describe disposable diapers having dual cuffs, including gasketing cuffs and barrier cuffs.

The fasteners 22 preferably maintain the front waist region 30 and the back waist region 32 in a configuration so as to provide lateral tensions about the circumference of the diaper 10 to hold the diaper 10 on the wearer. The fasteners 22 can be joined to the side connectors 24 by any attachment means known in the art; suitable attachment means are described above with respect to means for joining the outer casing 12 to the inner casing 14. The fasteners 22 can comprise surface fasteners such as tape tabs, hook and loop fastening components and/or hermaphroditic fastening components, although any other known fastening means are generally acceptable. In alternative embodiments, opposing sides of the article can be seamed or welded to form a pant. This allows the article to be used as a pull-on type diaper, a training pant and the like.

The side panels 24 are preferably elastic to provide a more comfortable and contouring fit by initially conformably fitting the diaper 10 to the wearer and sustaining this fit throughout the time of wear well past when the diaper 10 has been loaded with exudates since the side panels 24 can expand and contract. While the diaper 10 of the present invention preferably has the side panels 24 disposed in the back waist region 32, the diaper 10 can be provided with side connectors disposed in the front waist region 30 or both. The side panels 24 can be constructed in any suitable configurations. Examples of diapers with elasticized side connectors are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; the herein before referenced U.S. Pat. No. 5,151,092 issued to Buell on September 9, 1992; U.S. Pat. No. 5, 221,274 issued to Buell on June 22, 1993; U.S. Patent No. 5,669,897 issued to LaVon, et al. on September 23, 1997 entitled "Absorbent Articles Providing Sustained Dynamic Fit"; and U.S. Patent No. 6,004,306 entitled "Absorbent Article With Multi-Directional Extensible Side Panels" issued to Robles et al. on December 21, 1999.

The side panel 24 is preferably not susceptible to an electromagnetic field.

Referring to Figure 3, in preferred embodiments of the present invention, the side panel 24 comprises a three-layer-laminate construction including an elastic film 40 disposed between 2 non-woven layers, an outer layer 42 and an inner layer 44. As shown in Figure 3, the non-woven layer 48 of the outer casing 12 is facing the inner non-woven layer 44 of the side panel 24. However, it should be noted, that it is not necessary that the non-woven layers 44 and 48 face each other, and any other facing combinations, for example, a non-woven facing a film or a film facing a film can be included within the scope of the present invention.

In preferred embodiments of the present invention, the side panel 24 is preferably joined to the outer casing 12 directly above the absorbent core 16 by a fusion bond 50, which preferably does not extend into an outer surface 52 of the side panel 24 so as the tactile property of a receptor area 53 of the outer surface 52 is substantially equal to the tactile property of the outer surface 52 surrounding the receptor area 53. The term "receptor area" means herein a portion of the outer surface 52 disposed directly above the fusion bond 50. The term "substantially equal" means herein that the receptor area 53 and the outer surface 52 are free of raspy, rough, rigid protuberances or ruptures that can be sensed by touching by hand.

The outer surface 52 can comprise any suitable number of the receptor areas 53. The receptor area 53 can have any suitable shape or configuration in the x-y direction corresponding with the shape or configuration of the fusion bond 50 in the x-y direction. For example, if the fusion bond 50 is a round spot in the x-y direction, the receptor area 53 can be a round spot on the outer surface 52; if the fusion bond has an elongated shape in the x-y direction, the receptor area can have also an elongated shape in the x-y direction on the outer surface 52.

The term " fusion bond" herein refers to the joining of one material to another material to effect fusion of one material to another material. The fusion can be induced by a thermal interaction or a chemical reaction. With respect to the thermal interaction, the adjacent surfaces of the materials to be bonded are heat melted and held in an intimate contact to cool so as to form a bond. With respect to the chemical reaction, the adjacent surfaces of the materials to be bonded are held in an intimate contact to enable the reactive species to bridge the interface and to react to form a bond.

The term "material" refers herein to any substrate material including a film, a non-woven, a foam, a scrim, a laminate, a non-woven impregnated with thermoplastic materials, and the like, and any combination thereof.

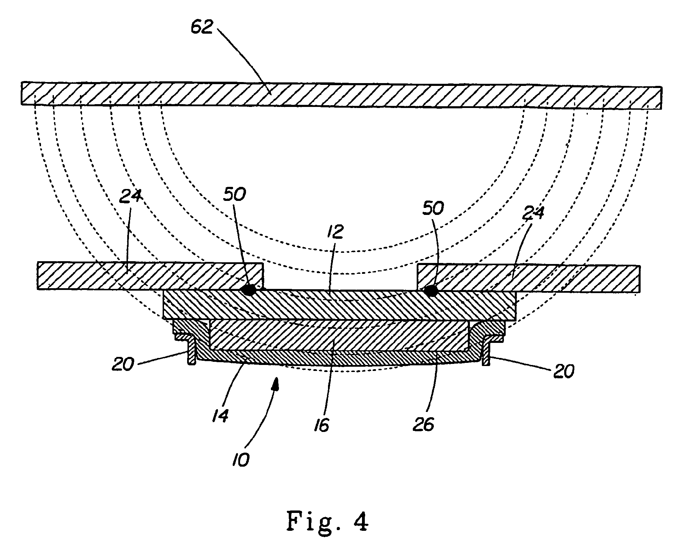

Figure 4 schematically illustrates a cross-sectional view of one embodiment of a method 58 of the present invention to produce a fusion bond 50 between one or more side panels 24 and the outer casing 12 of the disposable absorbent article 10, wherein the fusion bond 50 is located directly above the absorbent core 16. As shown in Figure 4, the article 10 can be subjected to an electromagnetic field 60, which can be provided by any suitable source 62 capable of generating the desired electromagnetic field 60. The electromagnetic field 60 can be generated in any region of the electromagnetic spectrum extending from the X-ray frequency region to the radio frequency region (from greater than about 10¹⁴ MHz to less than about 0.1 MHz), depending on the type of the fusion bond desired. For example, the electromagnetic field 60 generated above the visible frequency region of the electromagnetic spectrum (i.e., in the direction of increased frequency, in MHz, from the ultraviolet frequency region through the X-ray frequency region) can generally produce a chemical fusion bond, for instance, electron beam energy (a form of X-ray energy) can be used to promote chemical reactions (such as crosslinking) in polyethylene. The electromagnetic field 60 in the visible frequency region of the electromagnetic spectrum and below (i.e., in the direction of decreased frequency, in MHz, from the visible frequency region through the radio frequency region) can generally promote a thermal fusion bond. The above regions of the electromagnetic spectrum can be provided by any suitable commercial or specifically designed energy source; the energy source is not a part of the invention.

In one embodiment of the present invention, the electromagnetic field 60 can be generated in the radio frequency region and the microwave frequency region of the electromagnetic spectrum by the source 62. In the radio frequency region, the source 62, can be, for example, the CODACO Cure-On-Demand Adhesive System available from Ameritherm Inc. of New York. The frequency of the electromagnetic field 60 in the radio frequency region and the microwave frequency region can range from about 1.0 MHz to about 30,000 MHz. Particularly, in the radio frequency region, the frequency can range from about 1.0 MHz to about 300 MHz; and in the microwave frequency region, the frequency can range from about 300 MHz to about 30,000 MHz. Preferably, due to the practical considerations (for example, cost of energy sources which are readily available in a particular frequency range, equipment electromagnetic shielding requirements for a particular frequency range, and the like), in the preferred embodiment of the present invention, in the radio frequency region, the electro magnetic field 60 can range from about 1.0 MHz to about 180 MHz; and in the microwave frequency region, the electro magnetic field 60 can range from about 300 MHz to about 5,000 MHz or from about 915 MHz to about 2,450 MHz.

The power requirements of the equipment for generating the electromagnetic field 60 for bonding materials used in production of absorbent disposable articles will be a function of the thermo physical properties of the bonded materials, the desired bond area and the desired process speed or duration of exposure to the electromagnetic field, as well as the efficiency of generating the radio frequency field within the receptive material.

Referring to Figure 4, the fusion bond 50 can be provided by use of a receptor 54 susceptible to an electromagnetic field 60. The term "receptor susceptible to an electromagnetic field" means herein any material or a substance that becomes sufficiently heated or chemically active during exposure to an electromagnetic field having a sufficient energy to affect sufficient heat or chemical activity.

Referring to Figure 4, the receptor 54 can comprise various materials susceptible to specific regions of the electromagnetic spectrum depending on specific material properties. The term "susceptible to an electromagnetic field," refers herein to any material or substance that upon exposure to an electromagnetic field is affected such that a thermal or chemical reaction occurs inside the material. The fundamental material properties defining the magnitude of susceptibility to different regions of the electromagnetic spectrum are described in technical literature and are known to one skilled in the art. The receptor 54 can be in any suitable form, such as a solid, liquid, emulsion, dispersion, slurry, suspension or any combination thereof.

For example, in the radio frequency region and in the microwave frequency region, the material susceptibility in the above regions of the electromagnetic spectrum can be defined by such fundamental material property as the dielectric loss factor (DLF) of a specific material. Materials having a DLF of less than about 0.05 are considered inactive or not susceptible to the electromagnetic field in both the radio and microwave frequency regions. Materials having a DLF ranging from 0.05 to 0.10 are considered as weakly-active or weakly-susceptible to the electromagnetic field in both the radio and microwave frequency regions. Materials having a DLF ranging from 0.10 to 0.20 are considered active, and have good susceptibility to the electromagnetic field in both the radio and microwave frequency regions. And materials having DLF greater than 0.20 are considered very active or very susceptible to the electromagnetic field in both the radio and microwave frequency regions.

Materials susceptible to the radio frequency region and the microwave region can include, for example, the following materials: polymers based on vinyl chloride, vinylidene chloride, vinyl alcohol, vinyl ketones, vinylene carbonate, vinyl carbonates, vinyl esters, amides, imides, esters, carbonates, sulphones, sulfoxides, phosphates, phosphonates, polyurethanes, lactones, lactames, and phenoxys and salts thereof; thermoplastic block copolymers containing polyamides, polyimides, polysulphones, polyesters, polycarbonates; cellulose, and cellulose derivatives, for example, esters, ethers, carboxylates, alcoxylates, and nitrates and salts thereof; ethylene carbon monoxide containing polymers and interpolymers; acrylates and acid modified acrylates and salts thereof; -olefins containing olefinic unsaturated carboxylic acid and esters thereof and salts thereof; thermoplastic ionic starch polymers and salts thereof; proteins; epoxy resins; above said polymers incorporating polar solvents and plasticizers, for example but not limited to, water, glycerol, diglycerol, sorbitol, polyglycerols and mixtures thereof; the above said polymers incorporating organic and inorganic salts for example but not limited to, sodium chloride, potassium chloride, ammonium chloride, calcium chloride, lithium chloride, sodium sterate, lithium acetate, combinations and mixtures thereof.

In one embodiment of the present invention utilizing the radio frequency region of the electromagnetic spectrum, the receptor 54 can be any material having a DLF greater than about 0.15 at a temperature ranging from 20°C to greater than 170°C. In another embodiment of the present invention utilizing the microwave region of the electromagnetic spectrum, the receptor 54 can be any material having a DLF greater than about 0.10 at a temperature ranging from 20°C to greater than 170°C. It should be noted that the inventors selected the above range of temperature, from 20°C to greater than 170°C, based on the melting temperature of the preferred substrate materials to be used with the receptor 54 in the present invention; however, any suitable temperature (above or below the above temperature range) can be selected depending on the melting temperature of the bonded substrates.

In a preferred embodiment of the present invention, the receptor 54 can be any suitable receptor. For example, the receptor 54 can be one of the cure-on-demand adhesives available from Ameritherm Inc. of New York. Suitably, the receptor 54, when exposed to an electromagnetic field in the radio frequency region and the microwave frequency region, can have an activation time of less than about 1.0 second, or less than about 0.30 seconds, or less than about 0.15 seconds. The "activation time" means herein the time, during which the receptor 54 is exposed to the electromagnetic field.

The receptor 54 can be provided to the bonding surfaces by any suitable devices, including any typical hot melt adhesive dispensing equipment, spiral applicators, melt-blown applicators, beads applicators, and various nozzles suitable, for example, for use with UFD applicators available from L T. W. Dynatec of Tennessee.

In order to provide the fusion bond 50, which does not extend into an outer surface 52 of the side panel 24 so as the tactile property of the receptor area 53 of the outer surface 52 is substantially equal to the tactile property of the outer surface 52 surrounding the receptor area 53, the expansion of the fusion bond 50 into the outer casing 12 and the side panel 24 can be controlled by combination of the following factors: (a) the power supplied to generate the electromagnetic field 60 at a desired frequency; (b) the mass of the receptor 54; and (c) the duration of exposure of the receptor 54 to the electromagnetic field 60. The fusion bond 50 can be provided by the method of the present invention, wherein the receptor 54 is exposed to the electromagnetic field 60 for a time of less than about 1 second, preferably less than about 0.5 seconds, and more preferably less than about 0.3 seconds. The exposure time can also be suitably greater than about 0.1 seconds.

## Claims

1. A disposable absorbent article (10) comprising:
a) a first component;
b) a second component underlying the first component;
c) a third component underlying the second component; and
d) at least one fusion bond (50) disposed between the first component and the second component,
**characterized in that**
the fusion bond (50) being disposed directly above the third component and bonding at least partially the first component to the second component, the fusion bond comprising a receptor (54) susceptible to an electromagnetic field, and **in that** the first component is a side panel (24), a side connector or an car of the disposable absorbent article (10) and the second component is an outer casing (12) of the disposable absorbent article (10) and the third component is an absorbent core (16) of the disposable absorbent article (10).

2. The article according to any of the preceding claims wherein the receptor is susceptible to the electromagnetic field having frequency from 1.0 MHz to 30,000 MHz.

3. The article according to any of the preceding claims wherein the receptor is susceptible to the electromagnetic field having frequency from 1.0 MHz to 180 MHz.

4. The article according to any of the preceding claims wherein the receptor is susceptible to the electromagnetic field having frequency from 300 MHz to 5,000 MHz.

5. The article according to any of the preceding claims wherein the receptor is susceptible to the electromagnetic field having frequency from 915 MHz to 2,450 MHz.

6. The article according to any of the preceding claims wherein the receptor is selected from the group consisting of a solid, a liquid, an emulsion, a dispersion, a slurry, a suspension and any combination thereof.

7. The article according to any of the preceding claims wherein the first component comprises one or more layers of materials selected from the group consisting of a film, a non-woven, a foam, a scrim, a laminate, a non-woven impregnated with thermoplastic materials, an elastic material, and any combination thereof.

8. The article according to any of the preceding claims wherein the second component comprises one or more layers of materials selected from the group consisting of consisting of a film, a non-woven, a foam, a scrim, a laminate, a non-woven impregnated with thermoplastic materials, an elastic material, and any combination thereof.

9. A method of bonding components of a disposable absorbent article (10), the method comprising the steps of:
a) providing a disposable absorbent article (10) including:
i. a first component;
ii. a second component underlying the first component;
iii. a third component underlying the second component; and
iv. a receptor susceptible (54) to an electromagnetic field and disposed between the first component and the second component, **characterized in that**
the first component and the second component being at least partially in a contacting relation through the receptor; and **in that** the first component of the article (10); is a side panel (24), a side connector or an ear of the disposable absorbent article (10); and and the second, component of the article is an outer casing (12) of the disposable absorbent article (10); and the third component of the article (10) is an absorbent core (16) of the disposable absorbent article (10).
(b) exposing the receptor (54) to the electromagnetic field for a time period sufficient to heat the receptor (54) so as at least partially to melt and fuse the first and second components into each other, and
(c) while maintaining the contacting relation of the first and second components, cooling the first and second components to solidify a fusion bond between the first and the second components.

10. The method according to Claim 9 wherein the receptor is susceptible to the electromagnetic field having frequency from 1.0 MHz to 30,000 MHz.

11. The method according to Claim 9 wherein the receptor is susceptible to the electromagnetic field having frequency from 1.0 MHz to 180 MHz.

12. The method according to Claim 9 wherein the receptor is susceptible to the electromagnetic field having frequency from 300 MHz to 5,000 MHz.

13. The method according to Claim 9 wherein the receptor is susceptible to the electromagnetic field having frequency from 915 MHz to 2,450 MHz.

14. The method according to Claim 9 wherein the receptor (54) is selected from the group consisting of a solid, a liquid, an emulsion, a dispersion, a slurry, a suspension and any combination thereof.

## Patentansprüche

1. Einwegabsorptionsartikel (10), umfassend:
a) einen ersten Bestandteil;
b) einen zweiten Bestandteil, der unter dem ersten Bestandteil liegt;
c) einen dritten Bestandteil, der unter dem zweiten Bestandteil liegt; und
d) mindestens eine Schmelzverbindung (50), die zwischen dem ersten Bestandteil und dem zweiten Bestandteil angeordnet ist,
**dadurch gekennzeichnet, dass**
die Schmelzverbindung (50) direkt über dem dritten Bestandteil angeordnet ist und mindestens teilweise den ersten Bestandteil mit dem zweiten Bestandteil verbindet, wobei die Schmelzverbindung einen Rezeptor (54) umfasst, der für ein elektromagnetisches Feld empfänglich ist, und dadurch, dass der erste Bestandteil ein Seitenfeld (24), ein Seitenverbindungsstück oder ein Flügel des Einwegabsorptionsartikels (10) ist und der zweite Bestandteil ein äußeres Gehäuse (12) des Einwegabsorptionsartikels (10) ist und der dritte Bestandteil ein Absorptionskern (16) des Einwegabsorptionsartikels (10) ist.

2. Artikel nach einem der vorstehenden Ansprüche, wobei der Rezeptor für das elektromagnetische Feld mit einer Frequenz von 1,0 MHz bis 30.000 MHz empfänglich ist.

3. Artikel nach einem der vorstehenden Ansprüche, wobei der Rezeptor für das elektromagnetische Feld mit einer Frequenz von 1,0 MHz bis 180 MHz empfänglich ist.

4. Artikel nach einem der vorstehenden Ansprüche, wobei der Rezeptor für das elektromagnetische Feld mit einer Frequenz von 300 MHz bis 5.000 MHz empfänglich ist.

5. Artikel nach einem der vorstehenden Ansprüche, wobei der Rezeptor für das elektromagnetische Feld mit einer Frequenz von 915 MHz bis 2.450 MHz empfänglich ist.

6. Artikel nach einem der vorstehenden Ansprüche, wobei der Rezeptor ausgewählt ist aus der Gruppe bestehend aus einem Feststoff, einer Flüssigkeit, einer Emulsion, einer Dispersion, einem Brei, einer Suspension und einer Kombination davon.

7. Artikel nach einem der vorstehenden Ansprüche, wobei der erste Bestandteil eine oder mehrere Schichten von Materialien umfasst, die ausgewählt sind aus der Gruppe bestehend aus einer Folie, einem Vliesstoff, einem Schaumstoff, einem Gitterstoff, einem Schichtstoff, einem Vliesstoff, der mit thermoplastischen Materialien getränkt ist, einem elastischen Material und einer Kombination davon.

8. Artikel nach einem der vorstehenden Ansprüche, wobei der zweite Bestandteil eine oder mehrere Schichten von Materialien umfasst, die ausgewählt sind aus der Gruppe bestehend aus einer Folie, einem Vliesstoff, einem Schaumstoff, einem Gitterstoff, einem Schichtstoff, einem Vliesstoff, der mit thermoplastischen Materialien getränkt ist, einem elastischen Material und einer Kombination davon.

9. Verfahren zum Verbinden von Bestandteilen eines Einwegabsorptionsartikels (10), wobei das Verfahren die folgenden Schritte umfasst:
a) Bereitstellen eines Einwegabsorptionsartikels (10), umfassend:
i. einen ersten Bestandteil;
ii. einen zweiten Bestandteil, der unter dem ersten Bestandteil liegt;
iii. einen dritten Bestandteil, der unter dem zweiten Bestandteil liegt; und
iv. einen Rezeptor (54), der für ein elektromagnetisches Feld empfänglich ist und zwischen dem ersten Bestandteil und dem zweiten Bestandteil angeordnet ist,
**dadurch gekennzeichnet, dass**
der erste Bestandteil und der zweite Bestandteil durch den Rezeptor mindestens teilweise in einer Kontaktbeziehung sind; und dadurch, dass der erste Bestandteil des Artikels (10) ein Seitenfeld (24), ein Seitenverbindungsstück oder ein Flügel des Einwegabsorptionsartikels (10) ist; und der zweite Bestandteil des Artikels ein äußeres Gehäuse (12) des Einwegabsorptionsartikels (10) ist; und der dritte Bestandteil des Artikels (10) ein Absorptionskern (16) des Einwegabsorptionsartikels (10) ist(;)
b) Aussetzen des Rezeptors (54) gegenüber dem elektromagnetischen Feld für einen ausreichenden Zeitraum, um den Rezeptor (54) so zu erwärmen, dass er mindestens teilweise schmilzt und den ersten und den zweiten Bestandteil miteinander verbindet; und
c) unter Aufrechterhaltung der Kontaktbeziehung zwischen dem ersten und dem zweiten Bestandteil, Kühlen des ersten und zweiten Bestandteils, um eine Schmelzverbindung zwischen dem ersten und dem zweiten Bestandteil zu verfestigen.

10. Verfahren nach Anspruch 9, wobei der Rezeptor für das elektromagnetische Feld mit einer Frequenz von 1,0 MHz bis 30.000 MHz empfänglich ist.

11. Verfahren nach Anspruch 9, wobei der Rezeptor für das elektromagnetische Feld mit einer Frequenz von 1,0 MHz bis 180 MHz empfänglich ist.

12. Verfahren nach Anspruch 9, wobei der Rezeptor für das elektromagnetische Feld mit einer Frequenz von 300 MHz bis 5.000 MHz empfänglich ist.

13. Verfahren nach Anspruch 9, wobei der Rezeptor für das elektromagnetische Feld mit einer Frequenz von 915 MHz bis 2.450 MHz empfänglich ist.

14. Verfahren nach Anspruch 9, wobei der Rezeptor (54) ausgewählt ist aus der Gruppe bestehend aus einem Feststoff, einer Flüssigkeit, einer Emulsion, einer Dispersion, einem Brei, einer Suspension und einer Kombination davon.

## Revendications

1. Article absorbant jetable (10) comprenant :
a) un premier composant ;
b) un deuxième composant qui se trouve sous le premier composant ;
c) un troisième composant qui se trouve sous le deuxième composant ; et
d) au moins une liaison par fusion (50) disposée entre le premier composant et le deuxième composant,
**caractérisé en ce que**
la liaison par fusion (50) est disposée directement au-dessus du troisième composant et lie au moins partiellement le premier composant au deuxième composant, la liaison par fusion comprenant un récepteur (54) sensible à un champ électromagnétique, et **en ce que** le premier composant est un pan latéral (24), un connecteur latéral ou une patte de l'article absorbant jetable (10) et le deuxième composant est un revêtement externe (12) de l'article absorbant jetable (10) et le troisième composant est une âme absorbante (16) de l'article absorbant jetable (10).

2. Article selon l'une quelconque des revendications précédentes, dans lequel le récepteur est sensible au champ électromagnétique ayant une fréquence allant de 1,0 MHz à 30 000 MHz.

3. Article selon l'une quelconque des revendications précédentes, dans lequel le récepteur est sensible au champ électromagnétique ayant une fréquence allant de 1,0 MHz à 180 MHz.

4. Article selon l'une quelconque des revendications précédentes, dans lequel le récepteur est sensible au champ électromagnétique ayant une fréquence allant de 300 MHz à 5000 MHz.

5. Article selon l'une quelconque des revendications précédentes, dans lequel le récepteur est sensible au champ électromagnétique ayant une fréquence allant de 915 MHz à 2450 MHz.

6. Article selon l'une quelconque des revendications précédentes, dans lequel le récepteur est choisi dans le groupe constitué d'un solide, un liquide, une émulsion, une dispersion, une bouillie, une suspension et n'importe quelle combinaison de ceux-ci.

7. Article selon l'une quelconque des revendications précédentes, dans lequel le premier composant comprend une ou plusieurs couches de matériaux choisis dans le groupe constitué d'un film, un non-tissé, une mousse, une mousseline, un stratifié, un non-tissé imprégné de matériaux thermoplastiques, un matériau élastique, et n'importe quelle combinaison de ceux-ci.

8. Article selon l'une quelconque des revendications précédentes, dans lequel le deuxième composant comprend une ou plusieurs couches de matériaux choisis dans le groupe constitué d'un film, un non-tissé, une mousse, une mousseline, un stratifié, un non-tissé imprégné de matériaux thermoplastiques, un matériau élastique, et n'importe quelle combinaison de ceux-ci.

9. Procédé de liaison de composants d'un article absorbant jetable (10), le procédé comprenant les étapes consistant à :
a) fournir un article absorbant jetable (10) incluant :
i. un premier composant ;
ii. un deuxième composant qui se trouve sous le premier composant ;
iii. un troisième composant qui se trouve sous le deuxième composant ; et
iv. un récepteur sensible (54) à un champ électromagnétique et disposé entre le premier composant et le deuxième composant,
**caractérisé en ce que**
le premier composant et le deuxième composant sont au moins partiellement dans une relation de mise en contact via le récepteur ; et **en ce que** le premier composant de l'article (10) est un pan latéral (24), un connecteur latéral ou une patte de l'article absorbant jetable (10) ; et le deuxième composant de l'article est un revêtement externe (12) de l'article absorbant jetable (10) ; et le troisième composant de l'article (10) est une âme absorbante (16) de l'article absorbant jetable (10).
b) exposer le récepteur (54) au champ électromagnétique pendant une période de temps suffisante pour chauffer le récepteur (54) de façon à fondre et fusionner au moins partiellement les premier et deuxième composants l'un dans l'autre ; et
c) tout en maintenant la relation de mise en contact des premier et deuxième composants, refroidir les premier et deuxième composants pour solidifier une liaison par fusion entre le premier composant et le deuxième composant.

10. Procédé selon la revendication 9, dans lequel le récepteur est sensible au champ électromagnétique ayant une fréquence allant de 1,0 MHz à 30 000 MHz.

11. Procédé selon la revendication 9, dans lequel le récepteur est sensible au champ électromagnétique ayant une fréquence allant de 1,0 MHz à 180 MHz.

12. Procédé selon la revendication 9, dans lequel le récepteur est sensible au champ électromagnétique ayant une fréquence allant de 300 MHz à 5000 MHz.

13. Procédé selon la revendication 9, dans lequel le récepteur est sensible au champ électromagnétique ayant une fréquence allant de 915 MHz à 2450 MHz.

14. Procédé selon la revendication 9, dans lequel le récepteur (54) est choisi dans le groupe constitué d'un solide, un liquide, une émulsion, une dispersion, une bouillie, une suspension et n'importe quelle combinaison de ceux-ci.
